Europäisches Patentamt

European Patent Office

Office européen des brevets

(19)

(11) Veröffentlichungsnummer: **0 100 521**
**B1**

(12) **EUROPÄISCHE PATENTSCHRIFT**

(45) Veröffentlichungstag der Patentschrift: **29.08.90**

(51) Int. Cl.⁵: **C 12 N 15/38,** A 61 K 39/245

(21) Anmeldenummer: **83107373.9**

(22) Anmeldetag: **27.07.83**

(54) Verfahren zur Herstellung eines Herpes-Antigens, dazu geeignetes Mittel sowie Verfahren zu seiner Herstellung und die Verwendung dieses Antigens.

(30) Priorität: **30.07.82 DE 3228501**

(43) Veröffentlichungstag der Anmeldung:
**15.02.84 Patentblatt 84/07**

(45) Bekanntmachung des Hinweises auf die
Patenterteilung:
**29.08.90 Patentblatt 90/35**

(84) Benannte Vertragsstaaten:
**AT BE CH DE FR GB IT LI NL SE**

(56) Entgegenhaltungen:
**EP-A-0 101 655**

SCIENCE, Band 203, 9. Februar 1979, Seiten
541-544, AAAS; L.W. ENQUIST et al.: "Cloning of
herpes simplex type 1 DNA fragments in a
bacteriophage lambda vector"

CHEMICAL ABSTRACTS, Band 96, Nr. 23, 7. Juni
1982, Seite 177, Nr. 194306e, Columbus, Ohio,
US; D.A. GALLOWAY u.a.: "Identification of
proteins encoded by a fragment of herpes
simplex virus type 2 DNA that has transforming
activity"

Die Akte enthält technische Angaben, die nach
dem Eingang der Anmeldung eingereicht
wurden und die nicht in dieser Patentschrift
enthalten sind.

(73) Patentinhaber: **BEHRINGWERKE
Aktiengesellschaft
Postfach 1140
D-3550 Marburg 1 (DE)**

(72) Erfinder: **Bröker, Michael, Dr.
Lindenweg 16
D-3550 Marburg 1 (DE)**
Erfinder: **Winnacker, Ernst-Ludwig, Prof. Dr.
Dall-Armistrasse 41a
D-8000 München 19 (DE)**
Erfinder: **Wolf, Hans, Prof. Dr.
Jos-Jägerhuber-Strasse 9
D-8130 Starnberg (DE)**

(74) Vertreter: **Meyer-Dulheuer, Karl-Hermann, Dr.
et al
HOECHST Aktiengesellschaft Postfach 3540
D-6200 Wiesbaden (DE)**

(56) References cited:
**CHEMICAL ABSTRACTS, Band 95, Nr. 19, 9.
November 1981, Seite 382, Nr. 165275t,
Columbus, Ohio, US; J.J. DOCHERTY u.a.:
"Identification of a virus-specific polypeptide
associated with a transforming fragment (BglII-
N) of herpes simplex virus type 2 DNA"**

Courier Press, Leamington Spa, England.

EP 0 100 521 B1

# EP 0 100 521 B1

(56) References cited:
PROCEEDINGS OF THE NATIONAL ACADEMY
OF SCIENCES USA, Band 79, November 1982,
Seiten 6612-6616; G.T.-Y. LEE u.a.: "Expression
of herpes simplex virus glycoprotein C from a
DNA fragment inserted into the thymidine
kinase gene of this virus"

SCIENCE, Band 218, 22. Oktober 1982, Seiten
381-384, AAAS; R.J. WATSON u.a.: "Herpes
simplex virus type-1 glycoprotein D gene:
nucleotide sequence and expression in
Escherichia coli"

## Beschreibung

Ein Protein, das die Bildung von Antikörpern gegen Herpes-Viren bewirkt, ist von großem Interesse für die Herstellung von Impfstoffen zur Prophylaxe und Therapie von Erkrankungen, die durch diese Viren vurursacht werden.

Untersuchungen in den letzten Jahren lassen vermuten, daß bestimmte Tumorarten durch Herpesviren (HV) hervorgerufen werden können. Deshalb ist an einen Impfstoff, beispielsweise gegen Herpes simplex Virus Typ 1 (HSV-1) oder Typ II (HSV-2) die Forderung zu stellen, daß dieser absolut frei sein muß von HSV—DNA (Desoxyribonucleinsäure) oder daß die virale DNA inaktiviert sein muß, um mögliche Transformationen auszuschließen.

Die konventionelle Herstellung eines diese Forderungen entsprechenden Impfstoffes würde aufwendige Reinigungsschritte verlangen.

Als antigene Komponenten von HSV wurden bisher einige Glycoproteine beschrieben, die in der Hülle des Virus integriert sind (sogenannte Hüllproteine) und denen Genorte auf der HSV—DNA zugeordnet werden, konnten.

Es bestand also die Aufgabe, ein Herpes-Antigen herzustellen, das frei von Nucleinsäure von Herpesviren ist und zur Herstellung eines Impfstoffes gegen Erkrankungen geeignet ist, die von Herpesviren verursacht werden.

Die Aufgabe wurde nun dadurch gelöst, daß ein Genomabschnitt (DNA) aus einem Herpes simplex-Virus isoliert und in einen Mirkoorganismus transferiert wird, in dem diese DNA repliziert und exprimiert wird. Im folgenden wird die Klonierung des Gemabschnittes, der für das Glycoprotein C (gC) codiert, beschrieben. Analog durchführbar ist das Verfahren auch für die anderen bereits beschriebenen Hüllproteine gA, gB, gD und gE sowie für jedes andere virale Protein.

In der Europäischen Patentanmeldung 0 013 828 ist ein Verfahren zur Herstellung eines Polypeptids mit antigenen Eigenschaften des Hapatitis B-Virus beschrieben, in welchem aus der DNA des Virus eine DNA-Sequenz, die für eine antigene Determinante des Virus codiert, mit DNA eines sogenannten "cloning vehicle" oder "Vektor" verbunden und diese kombinierte Desoxyribonucleinsäure in eine Wirtszelle eingeschleust wird, so daß diese das Polypeptid mit den antigenen Eigenschaften des Heptatitis B-Virus produziert.

Im folgenden wird ein zur Lösung der gestellten Aufgabe geeignetes Verfahren beschrieben. Es sind jedoch auch Varianten dieses Verfahrens für den erfindungsgemäßen Zweck verwendbar, beispielsweise solche mit anderen Vektor-Wirtszellen-Kombinationen.

## 1. Gewinnung Virus-spezifischer Nucleinsäure
### 1.1 Wahl des HSV-Stammes

Infektionen mit hohen Infektionsdosen führen in vielen, Virussystemen zur Bildung von defekten Virusgenomen. Deshalb wurde zur Vemeidung solcher Artefakte ein klonierter Virusstamm verwendet, mit dem in geringer Multiplizität (0,1 PFU/Zelle) Wirtszellen, beispielsweise Verozellen, infiziert wurden. Wir verwendeten HSV-1 Stamm F. Bei Arbeiten mit anderen Stämmen (mit z.B. cos oder McIntyre) wäre ebenso zu verfahren.

### 1.2 Isolierung der HSV—DNA

Zur Vermeidung von Verlusten, die bei einer Viruseinigung entstehen, wurde die virale DNA direkt aus dem Zell-Lysat gewonnen. Hierfür wurde die Zentrifugation im hoch auflösenden Dichtegradienten (KJ, NaJ) verwendet, die eine weitgehende Abtrennung der in der Schwimmdichte signifikant verschiedenen Zell-DNA von der viralen DNA ermöglichte. Die Zugabe von interkalierenden Farbstoffen, beispielsweise Ethidiumbromid, machte eine direkte Sichtkontrolle des Trennungsergebnisses und der Gewinung der viralen DNA möglich (Walbromers and Scheggat, Virology (1976) 74, 256—258).

## 2. Isolierung eines Genomabschnittes, der für ein antigenes Protein aus der Virushülle codiert

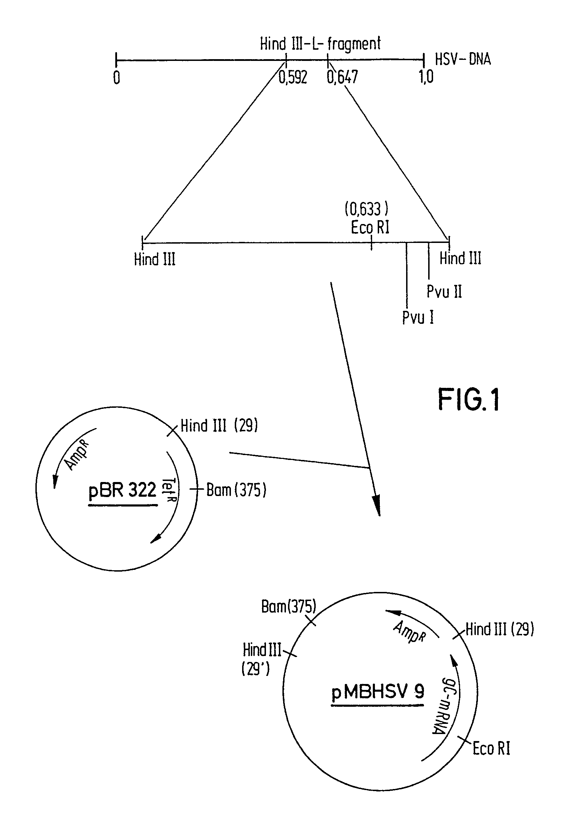

HSV1—DNA wurde mit Restriktionsendonucleasen hydrolysiert und die DNA-Fragmente in einem Agarosegel elektrophoretisch getrennt. Hierzu eignet sich die Nuclease HindIII da nach Verdauung der DNA mit diesem Enzym die vollständige Information für gC auf einem spezifischen Fragment lokalisiert ist (HindIII-L-Fragment). Das HindIII-L-Fragment (DNA-Abschnitt: 0,592—0,647 ca. 8200 bp, siehe auch Figur 1) wurde aus dem Agarosegel extrahiert (J. Langridge et al., Analyt. Biochem. *103*, 264—271 (1980). Ebenso läßt sich die DNA mit Methoden isolieren, wie sie H. O. Smith beschreibt (Meth. Enzymol. *65*, 371—380 (1980)).

## 3. Herstellung eines Plasmids, das den HSV-DNA-HindIII-L-Abschnitt besitzt und Transformation von Bakterien mit diesem rekombinanten Plasmid

Das HindIII-L-Fragment wurde in das Tetracyclingen (Tet-Gen) des Plasmids pBR 322 (F. Bolivar et al., Gene 2, 95 (1977)) ligiert (F. Bolivar and K. Backman, Meth. Enzymol *65*, 245 (1980) (Abb. 1)) und der *Escherichia coli* Stamm HB 101 mit diesem Plasmid transformiert.

Außer dem Plasmid pBR 322 können auch andere geeignete Vektoren verwendet werden wie zum Beispiel (pBR 327, pBR 328 oder pBR 329) (L. Covarrubias and F. Bolivar, Gene *17*, 79—89 (1982) oder pUC7, pUC8 oder pUC9 (J. Messing, Recombinant DNA, A.G. Walton, ed. 143—153 (1981), Elsevier Scientific Publishing Comp., Amsterdam) oder das Plasmid pUR 222 (U. Rüther et al., Nucl. Acids Res. *9* 4087—4098) (1981)).

Neben dem Stamm HB 101 sind andere Stämme von E. coli wie C600 (B. Bachmann, Bacteriol. Rev, *36*, 525—557) (1972)), RR1, SF8 oder SK1592 mit ähnlichen Methoden wie der von uns verwendeten transformierbar (S.L. Peacock et al., Biochim, Biophys. Acta 655, 243—250, (1981); M.G.M. Brown et al., FEMS Microbiol. Lett. *5* 219—222 (1979)).

Bei Transformationen von eukaryontischen Zellen könnte man als Vektoren zum Beispiel verwenden: Bovine Papilloma-Virus-DNA (N. Sarver et al., Mol. Cell. Biol. *1*, 486 bis 496 (1981); SV40 (J.T. Elder et al., Ann. Rev. Genetics *15*, 295—340 (1981) oder chimäre Plasmide wie pSG (R.M. Sendai-Goldin et al., Mol. Cell. Biol *1*, 743—752 (1981)).

4. Selektionierung geeigneter Klone

Die mit rekombinanten Plasmiden transformierten Zellen wurden auf Petrischalen mit L-Broth als Nährquelle unter Zusatz von Na-Ampicillin (50 mg/l) ausgespatelt und 1 Trag bei 37°C inkubiert. Klone, die sich als Ampicillin resistent und Tetracyclin sensitiv erwiesen, wurden gegen radioaktives HSV-1-HindIII-L-Fragment hybridisiert (E. Southern, Meth. Enzymol, *65*, 152—176 (1980)).

Positive Klone wurden in je 5,0 ml Flüssigkultur angezüchtet und die DNA isoliert (H.M. Goodman and R.J. MacDonald, Meth. Enzymol. *65* 75—90 (1980); D.S. Holmes and M. Quigley, Anal. Biochem *114*, 193—197 (1981)).

Durch Verdauung der rekombinierten Plasmide mit den Endonucleasen Hind III, Bam HI, Sal I, Pvul, Pvu II und Mst II und Vergleich mit der Restriktionskarte von HSV-1-Stamm COS, wurden Klone eindeutig identifiziert, die das Hind III-L-Fragment in die Hind III-Stelle des pBR 322 integriert hatten. Die Orientierung des L-Fragmentes und der gC-mRNA in pMB HSV 9 ist in Figur 1 dargestellt.

5. Verkürzung des Hind III-L-Fragmentes und Umklonierung des Fragments

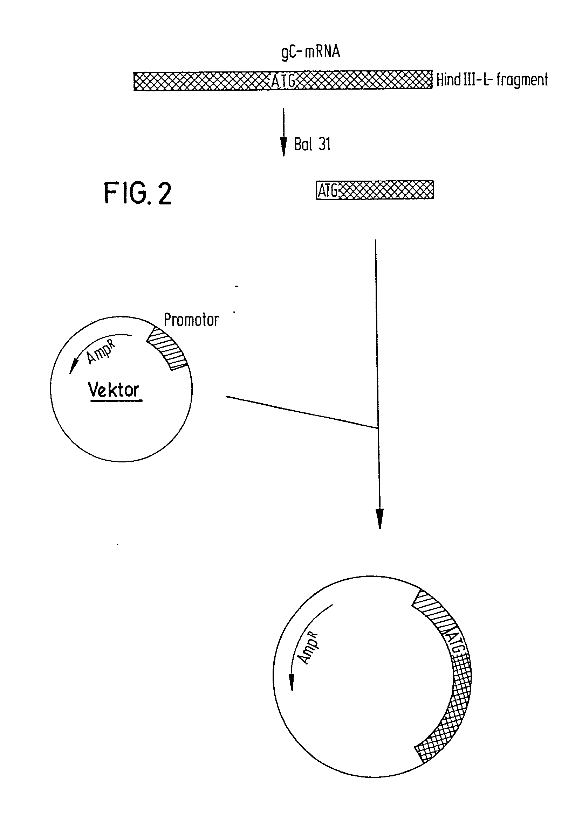

Das gC ist ein spätes (γ) Protein, dessen mRNA auf dem rechten Teil des Hind III-L-Fragmentes lokalisiert ist (Fink et al., J. Virol. in press, 1983). Da die gC-mRNA, die den Peptidanteil des gC codiert, vermutlich nicht gesplissen wird, kann die genomische Information in Prokaryonten exprimiert werden. Erstrebenswert ist es dabei, nicht nenötigte DNA-Anteile des Hind III-L-Fragmentes zur Synthese des gC zu entfernen. Dies kann unter anderem durch Verdauung der DNA mit geeigneten Endonucleasen erreicht werden. Durch Verdauung des Hind III-L-Fragmentes 5' endig vom Translationsstart mit der Exonuklease Bal 31 bis zum Translationsstart und Ligierung des verkürzten L-Fragmentes hinter einen effizienten Promoter (wie z.B. Trp-, Lac- oder Tac-Promotor) kann in prokaryontischen Systemen eine hohe Syntheserate des gC-Polypeptids erreicht werden (Figure 2).

Um eine Antikörperproduktion veranlassen zu können, wird eventuelle nicht das Gesamtprotein gC benötigt, sondern nur ein Teil des Proteins, das antigene Determinanten trägt. Zur Produktion solcher Polypeptide können klonierte DNA-Fragmente verwendet werden, bei denen mit Exonucleasen wie Bal31 in den codierten Bereich des gC hinein abgebaut worden ist oder Subfragmente des Hind III L-Fragmentes, wie beispielsweise das Eco/Hind-IL-Fragment.

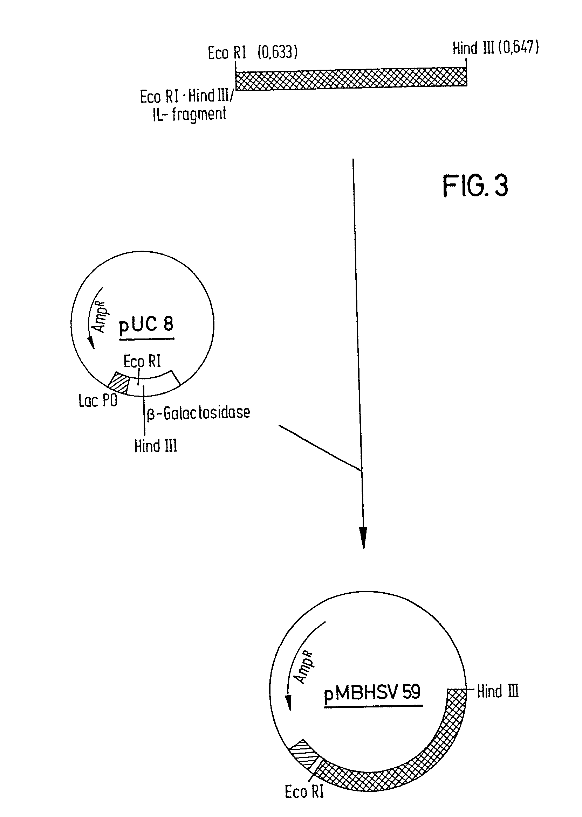

Die Ligierung des Eco/Hind-IL-Fragments in die ECo RI-Stelle hinter den lac UV 5 promotor in pUC 8 ermöglicht die Synthese eines Fusionsproteins mit den ersten NH$_2$-terminalen Aminosäuren der β-Galactosidase und dem größten Teil des gC-Polypeptids (Figur 3).

Eine Klonierung und Expression anderer antigener Glycoproteine von HSV als des gC kann analog den hier aufgeführten Vorgängen erfolgen.

Wenn ein Spleissen der entsprechenden mRNAs erfolgt kann die komplementäre DNA (cDNA) zur Expression der Glycoproteine verwendet werden, wobei kleine klonierte DNA-Fragmente wie das Eco/Hind-IL-Fragment, an das nur die gCmRNA von HSV-1 bindet, zur Selektionierung der entsprechenden mRNAs aus einem Lysat von mit HSV infizierten Zellen dienen können. Aber auch eine Klonierung der genomischen DNA ist in solchen Fällen denkbar, das auch das entsprechende, in Prokaryonten gebildete Genprodukt, trotz der hier nicht vorhandenen Splicing-Mechanismen ein Protein darstellen kann, das einige oder alle antigene Determinanten trägt. Sogar eine Verknüpfung von DNA-Fragmenten verschiedener Gene aus nur einem oder aus zwei unterschiedlichen Typen von Herpesviren und eine Expression solch eines Hybridproteins ist möglich.

Weiterhin ist eine Expression in eukaryontischen Zellen (Hefe, tierische Zellkulturen) möglich, vor allem dann, wenn eine Glycosilierung des Proteins wünschenswert ist.

Wegen seiner antigenen Eigenschaften ist ein solches Protein geeignet zur Gewinnung von Antiseren und/oder Impfstoffen, gegebenenfalls unter Verwendung von Adjuvantien und üblichen Hilfsstoffen.

Es kann auch für diagnostische Zwecke verwendet werden, beispielsweise zum Nachweis von Antikörpern gegen Herpes-Viren.

Antiseren gegen ein solches Protein können zum Nachweis von Herpes-Viren selbst dienen.

**Patentansprüche**

1. Verfahren zur Herstellung von Glykoprotein C des Herpes Simplex Virus Type 1 (HSV-1) oder immunogenen Teilen davon, dadurch gekennzeichnet, daß man das HindIII L-Fragment von HSV-1 oder für diese immunogenen Teile kodierenden Teilfragmente in Vektoren einbringt, mit diesen Vektoren pro- oder eukaryotische Zellen transformiert und in den Transformanten zur Expression bringt.

2. Verfahren nach Anspruch 1, dadurch gekennzeichnet, daß das EcoRI/HindIII IL-Fragment verwendet wird.

3. Verfahren nach Anspruch 1, dadurch gekennzeichnet, daß E.coli Zellen transformiert werden.

4. Verfahren nach Anspruch 1, dadurch gekennzeichnet, daß eukaryotische Zellen transformiert werden.

5. Polypeptid, erhältlich nach einem der Ansprüche 1 bis 4.

6. Verwendung eines Polypeptids nach

EP 0 100 521 B1

Anspruch 5 zur Gewinnung von Antiserum und/oder zur Herstellung eines Impfstoffs oder eines diagnostischen Mittels.

**Revendications**

1. Procédé pour la production de la glycoprotéine C du virus Herpes simplex type 1 (HSV-1) ou de parties immunogènes de celle-cil caractérisé en ce que l'on introduit dans des vecteurs le fragment HindIII-L de HSV-1 ou des fragments partiels codant pour ces parties immunogènes, on transforme à l'aide de ces vecteurs des cellules procaryotes ou eucaryotes et on réalise l'expression dans les transformants.

2. Procédé selon la revendication 1, caractérisé en ce que l'on utilise le fragment EcoRI/HindIII-IL.

3. Procédé selon la revendication 1, caractérisé en ce que l'on transforme des cellules de *E. coli*.

4. Procédé selon la revendication 1, caractérisé en ce que l'on transforme des cellules eucaryotes.

5. Polypeptide pouvant être obtenu selon l'une des revendications 1 à 4.

6. Utilisation d'un polypeptide selon la revendication 5, pour l'obtention d'antisérum et/ou pour la fabrication d'un vaccin ou d'un agent de diagnostic.

**Claims**

1. A process for the preparation of glycoprotein C of herpes simplex virus type 1 (HSV-1) or immunogenic components thereof, which comprises introducing the HindIII L fragment of HSV-1 or part fragments which encode these immunogenic components into vectors, and transforming pro- or eukaryotic cells with these vectors and causing them to be expressed in the transformants.

2. The process as claimed in claim 1, wherein the EcoRI/HindIII IL fragment is used.

3. The process as claimed in claim 1, wherein E. coli cells are transformed.

4. The process as claimed in claim 1, wherein eukaryotic cells are transformed.

5. A polypeptide obtainable as claimed in any one of claims 1 to 4.

6. The use of a polypeptide as claimed in claim 5 for isolating antiserum and/or for the preparation of a vaccine or a diagnostic agent.

FIG.1

FIG. 2

FIG. 3

Eco RI (0,633)    Hind III (0,647)

Eco RI · Hind III/
IL- fragment

pUC 8

Amp$_R$

Eco RI

Lac PO

β-Galactosidase

Hind III

pMBHSV 59

Amp$_R$

Hind III

Eco RI